(19) Europäisches Patentamt
European Patent Office
Office européen des brevets

(11) **EP 3 443 849 A1**

(12) **EUROPEAN PATENT APPLICATION**
published in accordance with Art. 153(4) EPC

(43) Date of publication:
**20.02.2019 Bulletin 2019/08**

(51) Int Cl.:
*A23L 33/145* [(2016.01)]     *A23L 7/109* [(2016.01)]
*A61K 36/064* [(2006.01)]     *A61P 3/10* [(2006.01)]

(21) Application number: **17782504.9**

(22) Date of filing: **14.04.2017**

(86) International application number:
**PCT/JP2017/015267**

(87) International publication number:
**WO 2017/179691 (19.10.2017 Gazette 2017/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **14.04.2016 JP 2016081494**

(71) Applicant: **TableMark Co., Ltd.**
**Tokyo 104-0045 (JP)**

(72) Inventors:
• **SHIMADA, Hiroki**
  **Tokyo 104-0045 (JP)**
• **MORISHIMA, Hiroki**
  **Tokyo 144-0042 (JP)**
• **HAISHIMA, Yoshitaka**
  **Tokyo 104-0045 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(54) **FOOD COMPOSITION OR COMBINATION FOR LOWERING BLOOD GLUCOSE LEVEL OR REDUCING GI VALUE**

(57)     The present invention relates to a food composition or a combination for lowering a blood glucose level or reducing a GI value. The food composition of the present invention is characterized by containing a yeast extract obtained from yeast belonging to the genus *Saccharomyces.* The combination of the present invention comprises the food composition of the present invention and a food composition containing grain as a main raw material. In one aspect, the food composition containing grain as a main raw material comprises a resistant starch.

Fig. 18

**Description**

TECHNICAL FIELD

The present invention relates to a food composition or a combination for lowering blood glucose levels or reducing GI values.

BACKGROUND ART

Control of Blood glucose level

The blood glucose level is the concentration of glucose in the blood. Human blood glucose levels are maintained within a very narrow range of normal levels by hormones such as insulin that lowers blood glucose levels, and glucagon, adrenaline, cortisol, and growth hormones that increase blood glucose levels. Although glucose *in vivo* is important as an energy source, a high glucose concentration causes glycosylation to damage microvessels, and thus is harmful for living organisms. Hence, the concentration (blood glucose level) is always maintained within a certain range by insulin and the like.

Animals have almost no defense mechanism against glucose elevations. When a blood glucose level increases, a hormone that regulates the level is only insulin. A failure of the only one regulatory mechanism causes the onset of hyperglycemia such as diabetes. It is extremely important to control *in vivo* blood glucose levels so as to prevent hyperglycemia.

Brewers' yeast is known to contain a substance having a property of reducing blood glucose levels. This substance is a quinoline derivative (molecular weight: 174) capable of binding to chromium (Japanese Examined Patent Publication No. H06-86379). Moreover, Eden et al., state in their study using rat adipocytes *in vitro* that a brewer's yeast-derived yeast extract enhances sugar uptake by insulin into adipocytes (N. Edens et al.,: J Nutr. 2002 Jun; 132(6): 1141-1148). However, data in the literature is merely *in vitro* data, and the effects when the extract is actually administered to animals remain unclarified.

Japanese Patent Laid-Open No. 2009-291076 discloses that a dry yeast extract obtained from *Saccharomyces cerevisiae* containing 2.5 wt% or more glutathione per dried cell exerted an effect of suppressing elevations in blood glucose levels in mice fed with high-fat powder feeds.

Regarding human foods, it is known that the GI values of whole diets are reduced via combination of a plurality of foods. Specifically, for example, Japanese Patent Laid-Open No. 2011-19402 describes a method for combining food materials using a plurality of food materials containing high GI food materials, wherein, on the basis of the ratio of the amount of carbohydrate of high GI food materials contained in the plurality of food materials to the total amount of carbohydrate, which is the sum of the amounts of carbohydrate of food materials composing the plurality of food materials, the proportion of each food material contained in the plurality of food materials to be combined is determined. This is based on a concept that high GI food materials and low GI food materials are combined to suppress the total amounts of carbohydrate. Regarding foods and beverages, the following knowledge has been obtained.

- The incremental area under the curve (IAUC) in the case of eating Udon noodle (thick Japanese noodles) or rice together with side dishes including eggs, vegetables, and fried egg plants with Chinese chili sauce is lower than that in the case of eating Udon noodle or rice alone (Matsushima M, et al.,: Glycative Stress Research. 2014; 1: 53-59); and
- Even in a case of fast food, through intake of a main dish in combination with side dishes, glycation stress can be alleviated (Kawabata A, et al.,: Glycative Stress Research. 2015; 2(2): 67-71).

However, a food composition excellent in function of controlling blood glucose levels (suppressing elevations in blood glucose level), and, taste and texture has never been provided.

Resistant starch

"Resistant starch" (RS) is a form of starch or a degradation product, which is resistant to the process of digestion by digestive enzymes and thus is not digested and absorbed within the small intestine of a healthy individual. RS is known to have an effect of improving intestinal environment, an effect of suppressing glucose elevations, a cholesterol-lowering effect, an effect of improving lipid metabolism, and the like. For example, Japanese Patent Laid-Open No. 2011-84674 describes Udon noodle containing a RS-rich starch. However, RS-containing foods such as noodles are powdery and dry, for example, and thus are not always satisfactory in terms of taste and texture.

CITATION LIST

PATENT LITERATURE

PTL 1: Japanese Patent Publication No. H6-86379
PTL 2: Japanese Patent Laid-Open No. 2009-291076
PTL 3: Japanese Patent Laid-Open No. 2011-19402
PTL 4: Japanese Patent Laid-Open No. 2011-84674 (JP Patent No. 4482611)

NON PATENT LITERATURE

NPL 1: N. Edens, et al.,: J Nutr. 2002 Jun; 132(6): 1141-1148.
NPL 2: Matsushima M, et al.,: Glycative Stress Research. 2014; 1: 53-59
NPL 3: Kawabata A, et al.,: Glycative Stress Research. 2015; 2(2): 67-71
NPL 4: Jenkins, D. J., et al.,: Am. J. Clin. Nutr., 1981; 34: 362-366

SUMMARY OF INVENTION

TECHNICAL PROBLEM

An object of the present invention is to provide a food composition or a combination for lowering blood glucose levels or reducing GI values.

As a result of intensive studies to achieve the above object, the present inventors have discovered that a yeast extract derived from yeast belonging to the genus *Saccharomyces* has a significant effect of lowering blood glucose levels and an effect of reducing GI values, and thus have conceived of a food composition of the present invention.

Furthermore, through combination of the food composition of the present invention containing a yeast extract derived from yeast belonging to the genus *Saccharomyces* with a food composition containing grain as a main raw material, the present inventors have succeeded in providing a combination of food compositions having a significant effect of lowering blood glucose levels and a significant effect of reducing GI values.

SOLUTION TO PROBLEM

The present invention encompasses, but is not limited to, the following aspects.

[Aspect 1] A food composition for lowering a blood glucose level, containing a yeast extract of yeast belonging to the genus *Saccharomyces.*

[Aspect 2] A food composition for reducing a GI value, containing a yeast extract of yeast belonging to the genus *Saccharomyces.*

[Aspect 3] The food composition according to aspect 1 or 2, containing the yeast extract in an amount of 0.001 wt% or more and 5.0 wt% or less in terms of the amount of the dry yeast extract.

[Aspect 4] The food composition according to any one of aspects 1 to 3, wherein the yeast belonging to the genus *Saccharomyces* belongs to *Saccharomyces cerevisiae.*

[Aspect 5] A method for enhancing an effect of lowering a blood glucose level of a food composition, comprising incorporating a yeast extract of yeast belonging to the genus *Saccharomyces* into the food composition.

[Aspect 6] A method for reducing a GI value of a food composition, comprising incorporating a yeast extract of yeast belonging to the genus *Saccharomyces* into the food composition.

[Aspect 7] A combination of the food composition according to any one of aspects 1 to 4 with a food composition containing grain as a main raw material.

[Aspect 8] The combination according to aspect 7, wherein the food composition containing grain as a main raw material is rice, a noodle, a rice cake, bread, Okonomi-yaki, Tako-yaki, Monja-yaki, pizza, tortilla, dim sum, a Chinese steamed bun, a Korean pancake, a cereal food or a confectionery.

[Aspect 9] The combination according to aspect 7 or 8, wherein the food composition containing grain as a main raw material is selected from the group consisting of Udon noodle, Hiyamugi, Soumen, Soba, pasta, Chinese noodles, and rice noodles.

[Aspect 10] The combination according to any one of aspects 7 to 9, wherein the food composition containing grain as a main raw material is Udon noodle.

[Aspect 11] The combination according to any one of aspects 7 to 10, wherein the food composition containing grain as a main raw material is produced by a method comprising a mixing step by reduced-pressure mixing.

[Aspect 12] The combination according to any one of aspects 7 to 11, wherein the food composition according to any one of aspects 1 to 4 is liquid.

[Aspect 13] The combination according to any one of aspects 7 to 12, wherein the food composition containing grain as a main raw material contains a resistant starch.

[Aspect 14] The combination according to aspect 13, wherein a content of the resistant starch is 0.6 wt% or more and less than 6 wt% of a total amount of powder raw materials.

[Aspect 15] The combination according to aspect 13 or 14, wherein the resistant starch is obtained by subjecting a high-amylose corn starch to acid treatment, and has a peak molecular weight of $6 \times 10^3$ or more and $4 \times 10^4$ or less.

[Aspect 16] The combination according to any one of aspects 7 to 15, used for lowering a blood glucose level.

[Aspect 17] The combination according to any one of aspects 7 to 15, used for reducing a GI value.

[Aspect 18] A method for enhancing an effect of lowering a blood glucose level of both of the food composition according to any one of claims 1 to 4 and a food composition containing grain as a main raw material, comprising combining the food compositions.

[Aspect 19] A method for reducing a GI value of both of the food composition according to any one of claims 1 to 4 and a food composition containing grain as a main raw material, comprising combining the food compositions.

[Aspect 20] Noodles for lowering a blood glucose level, which contain a resistant starch and are produced by a method comprising a mixing step by reduced-pressure mixing.

[Aspect 21] A method for reducing a GI value of noodles, comprising incorporating a resistant starch into the noodles and producing the noodles by a method comprising a mixing step by reduced-pressure mixing.

ADVANTAGEOUS EFFECTS OF INVENTION

The food composition of the present invention contains a yeast extract derived from yeast belonging to the genus *Saccharomyces,* so as to exert a significant effect of lowering blood glucose levels and a significant effect of reducing GI values.

Furthermore, the combination of the present invention exerts a significant effect of lowering blood glucose levels and a significant effect of reducing GI values through combination of the food composition of the present invention containing the yeast extract derived from yeast belonging to the genus *Saccharomyces* with the food composition containing grain as a main raw material. The combination of the present invention exerts significant effects through combination of a plurality of mechanisms for lowering blood glucose levels and reducing GI values. As an example, before the present invention, a low GI food composition containing a resistant starch used therein, such as noodles, was problematic in its dry texture, for example. In the combination of the present invention, adding the above yeast extract derived from yeast belonging to the genus *Saccharomyces* to Men-tsuyu (noodle dipping sauce), for example, makes it possible to reduce the amount of a resistant starch to be contained in noodles, and, to provide a noodle dish having a low GI value as the whole dish and good texture while contributing to the flavor and taste of the Tsuyu. Moreover, for example, adding the yeast extract derived from yeast belonging to the genus *Saccharomyces* to a sauce to be added to Okonomi-yaki enables to provide the Okonomi-yaki having a reduced GI value. Furthermore, a significant effect of reducing GI values was observed in a case of adding Shirodashi containing the yeast extract derived from yeast belonging to the genus *Saccharomyces* to cooked rice, than that in a case of adding Shirodashi containing no yeast extract.

BRIEF DESCRIPTION OF DRAWINGS

Fig. 1 shows changes over time in blood glucose level in the cases of "vacuum Udon noodle" × "No supplementation with yeast extract" in Example 1 (No. 1 in Table 1).

Fig. 2 shows changes over time in blood glucose level in the cases of "RS vacuum Udon noodle" × "No supplementation with yeast extract" in Example 1 (No. 2 in Table 1) (No. 1 in Table 4).

Fig. 3 shows changes over time in blood glucose level in the cases of "vacuum Udon noodle" × "supplementation with yeast extract NYP" in Example 1 (No. 3 in Table 1).

Fig. 4 shows changes over time in blood glucose level in the cases of "RS vacuum Udon noodle" × "supplementation with yeast extract NYP" in Example 1 (No. 4 in Table 1) (No.2 in Table 4).

Fig. 5 shows changes over time in blood glucose level in the cases of "atmospheric Udon noodle" × "No supplementation with yeast extract" in Example 1 (Comparative Example in Table 1).

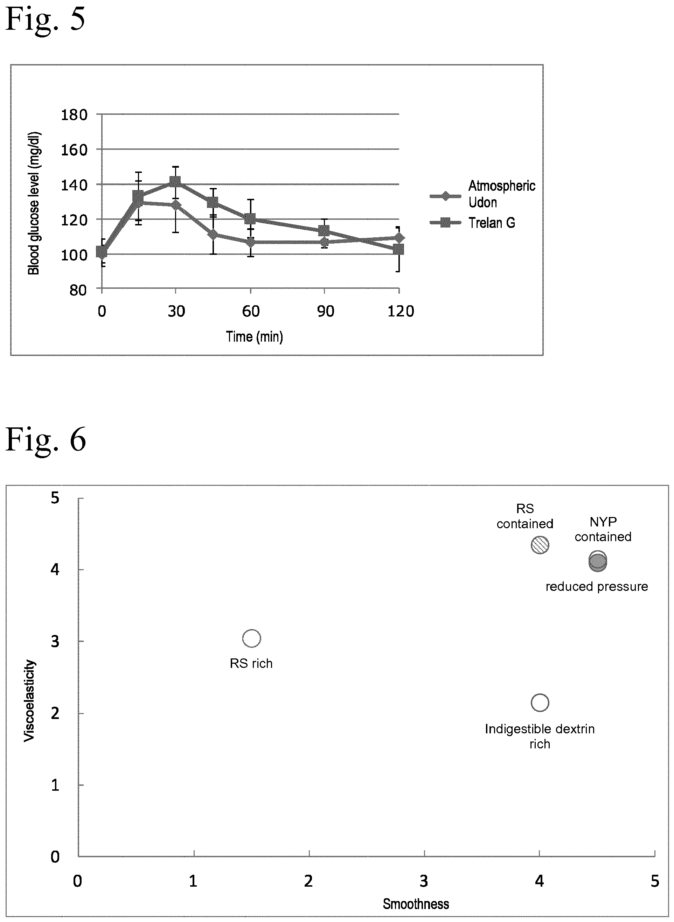

Fig. 6 shows the results obtained when ten well-trained panelists made sensory evaluations for the physical properties; that is, evaluation items of each Udon noodle noodle product (smoothness, viscosity, and elasticity) based on a 5-point evaluation system ranging from 1 (poor) to 5(good), and the scores marked by each panelist were averaged. Moreover, the average value of the thus obtained value of viscosity and value of elasticity was designated as viscoelasticity. In Fig. 6, viscoelasticity was plotted along the longitudinal axis, and smoothness was plotted along the horizontal axis. RS-rich vacuum Udon noodle (No. 1 in Table 3), indigestible dextrin-rich vacuum Udon noodle (No. 2 in Table 3), NYP-containing vacuum Udon noodle (No. 3 in Table 3), vacuum Udon noodle (No. 4 in Table 3, Comparative Example), RS-containing vacuum Udon noodle (No. 5).

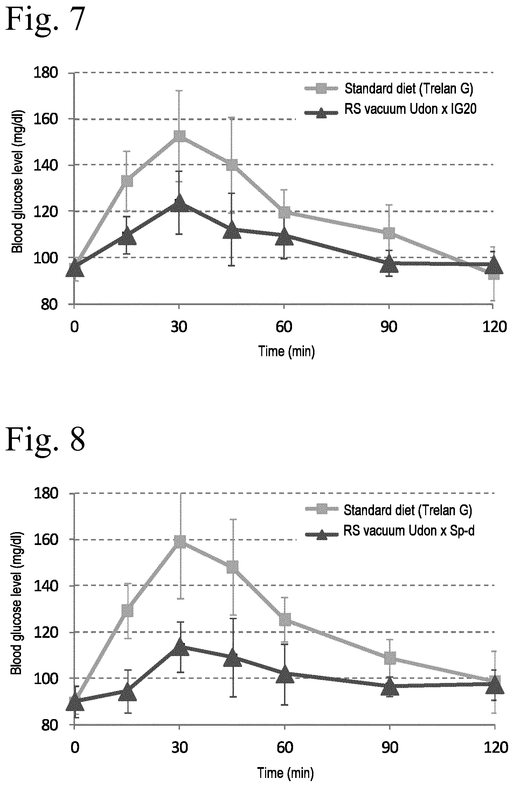

Fig. 7 shows changes over time in blood glucose level in the cases of "RS vacuum Udon noodle" × "Vertex (Registered Trademark) IG20" in Example 3 (No. 3 in Table 4).

Fig. 8 shows changes over time in blood glucose level in the cases of "RS vacuum Udon noodle" × "Sp-d" in Example 3 (No. 4 in Table 4).

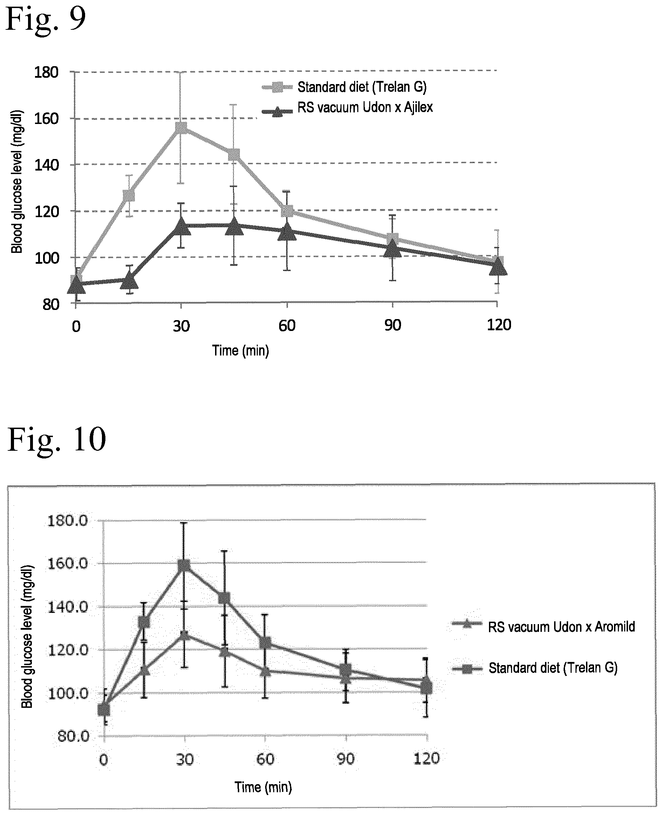

Fig. 9 shows changes over time in blood glucose level in the cases of "RS vacuum Udon noodle" × "Ajilex (Registered Trademark) LK" in Example 3 (No.5 in Table 4).

Fig. 10 shows changes over time in blood glucose level in the cases of "RS vacuum Udon noodle" × "Aromild (Registered Trademark)" in Example 3 (No. 6 in Table 4).

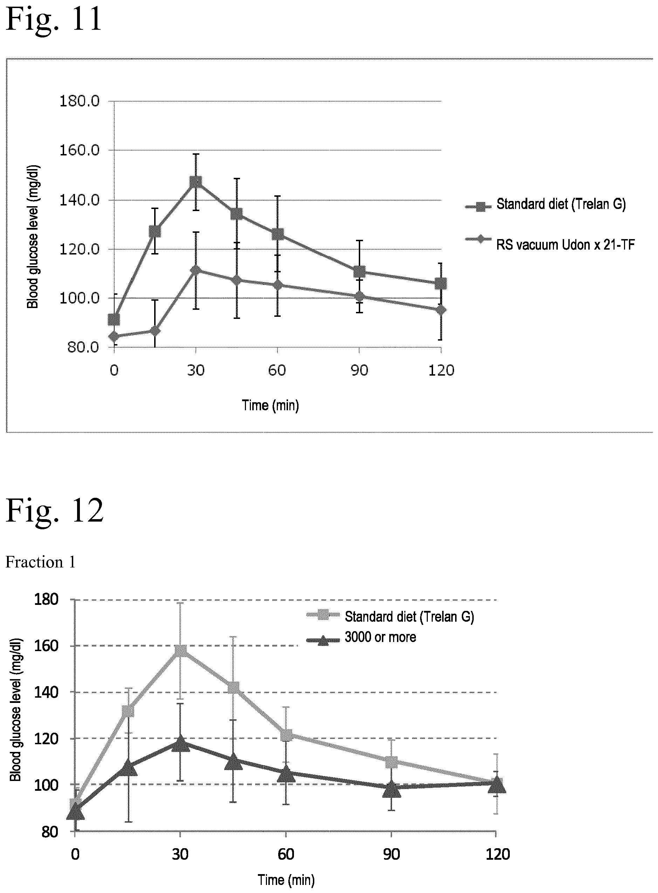

Fig. 11 shows changes over time in blood glucose level in the cases of "RS vacuum Udon noodle" × "Yeast extract 21-TF)" in Example 3 (No. 7 in Table 4).

Fig. 12 shows changes over time in blood glucose level in the case of Fraction 1 in Example 4.

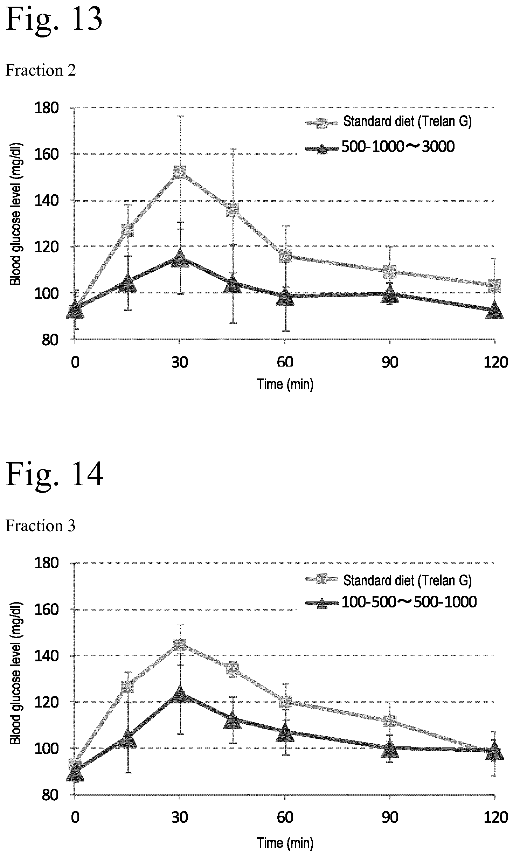

Fig. 13 shows changes over time in blood glucose level in the case of Fraction 2 in Example 4.

Fig. 14 shows changes over time in blood glucose level in the case of Fraction 3 in Example 4.

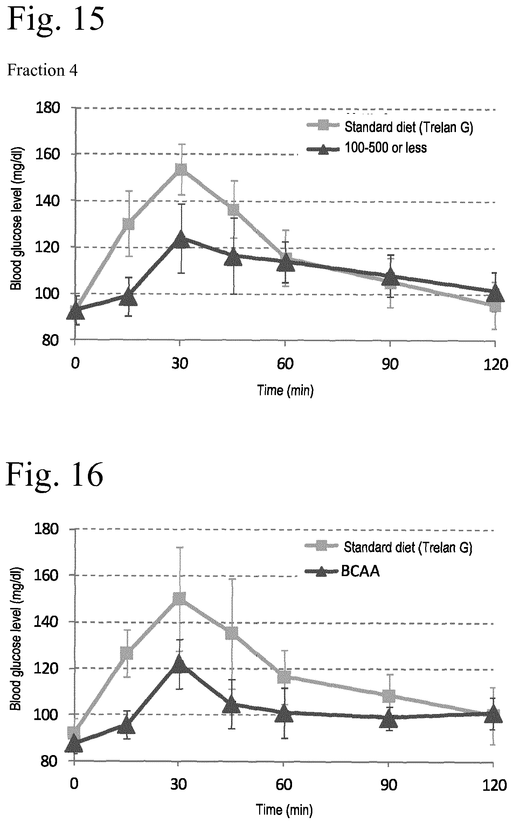

Fig. 15 shows changes over time in blood glucose level in the case of Fraction 4 in Example 4.

Fig. 16 shows changes over time in blood glucose level in the case of BCAA in Example 4.

Fig. 17 shows changes over time in blood glucose level in the case of nucleic acid in Example 4.

Fig. 18 shows changes over time in blood glucose level in the cases of test diet 1(the present invention) in Example 5 and test diet 2 (Comparative Example).

DESCRIPTION OF EMBODIMENTS

Food composition

The present invention relates to a food composition for lowering blood glucose levels, which contains a yeast extract of yeast belonging to the genus *Saccharomyces.*

The present invention also relates to a food composition for reducing GI values, which contains a yeast extract of yeast belonging to the genus *Saccharomyces.*

The yeast extract contained in the food composition of the present invention is a yeast extract of yeast belonging to the genus *Saccharomyces.* Such "yeast belonging to the genus *Saccharomyces*" belongs to the genus *Saccharomyces,* and is yeast that is mainly used for bread fermentation, and wine or beer brewing, for example. The yeast preferably belongs to *Saccharomyces cerevisiae* (also referred to as "bakers' yeast", or "budding yeast").

The term "yeast extract" refers to an extract obtained by extracting useful yeast ingredients by autodigestion or treatment with an enzyme, hot water or the like. In the present invention, a method for extracting a yeast extract is not particularly limited, for example, a yeast extract is obtained by extraction by a method such as hot water extraction, extraction by enzymatic degradation, or extraction by autodigestion. According to current food classification and food additive classification, yeast extracts are not classified in food additives, but classified in foods like soy sauces, dried Seaweed (Kombu) extracts, and the like.

Note that examples of the types of the yeast extract include a type containing, as major ingredients, amino acid or nucleic acid related substances, minerals, and vitamins, and containing nucleic acids in higher amounts, a type containing almost no nucleic acid, but containing amino acid alone, and a type containing both nucleic acid and amino acid in a well-balanced manner. In the case of the yeast extract of yeast belonging to the genus *Saccharomyces,* any type of the yeast extract was found to have an effect of lowering blood glucose levels (including an effect of reducing GI values) (Example 3).

Preferably, the yeast extract of the present invention is not derived from glutathione-rich yeast, but this is not limited thereto. The term "glutathione-rich yeast" refers to, for example, "*Saccharomyces cerevisiae* containing 2.5 wt% or more of glutathione per dried cell" as disclosed in Japanese Patent Laid-Open No. 2009-291076. Specific examples of such *Saccharomyces cerevisiae* include, *Saccharomyces cerevisiae* FERM P-19072 strain, *Saccharomyces cerevisiae* FERM P-19073 strain, and *Saccharomyces cerevisiae* FERM P-19074 strain (all strains were deposited under the accession numbers on October 18, 2002, with the International Patent Organism Depositary, the National Institute of Advanced Industrial Science and Technology) as disclosed in Japanese Patent Laid-Open No.2004-180509, their parent strains, or derivative strains obtained therefrom. The yeast extract of the present invention is preferably not derived from these glutathione-rich yeast strains. The term "glutathione content per dried cell" refers to the sum of the content of reduced glutathione and the content of oxidized glutathione. The glutathione content per dried cell of *Saccharomyces cerevisiae* to be used can be calculated by collecting cells from a culture solution of *Saccharomyces cerevisiae,* washing the cells with water, removing the medium components, suspending the resultant in distilled water, and then finding the concentration of solids in the suspension via heating and drying or freeze drying, as well as finding by HPLC analysis the contents of reduced glutathione and oxidized glutathione contained in the suspension heated at 85°C for 5 minutes.

The amount of a yeast extract to be added to a food composition is not particularly limited. Preferably, the upper limit of the amount in terms of dry yeast extract is 5.0 wt%, or 2.0 wt%, and the lower limit of the same is 0.001 wt%, 0.01 wt%, 0.05 wt%, or 0.1 wt% of the food composition. Persons skilled in the art can adequately adjust the amount of a yeast extract to be added depending on the type and the like of a food composition to which the yeast extract is added.

When a yeast extract obtained by extraction from *Saccharomyces cerevisiae* was fractionated, a fraction having the lower limit molecular weight of 500 and the upper limit molecular weight of 3000 was observed to specifically have an effect of lowering blood glucose levels (Example 4). The yeast extract of the present invention preferably contains a fraction included within a molecular weight range of 500 to 3000 of the yeast extract.

The expression "food composition for lowering a blood glucose level(s)" refers to a food composition, when the food composition of the present invention is taken as a main dish or when the same is taken together with a main dish, having an effect of lowering (or suppressing an elevation in) a blood glucose level compared with a case where no such food composition is taken. The blood glucose level is a concentration of glucose in the blood. In the case of humans, the fasting blood glucose level ranges from about 80 mg/dL to 120 mg/dL, and the blood glucose level slightly elevated after food intake, and reaches up to about 120-200 mg/dL after about 20 minutes to 40 minutes in a healthy subject. In the case of such a healthy subject, the level is decreased to the fasting blood glucose level within 120 minutes after food intake. In the present invention, the expression "blood glucose level is lowered (decreased) (or suppressing an elevation in blood glucose level)" refers to: the highest value of an elevated blood glucose level due to food intake is lowered; difference (the highest value of an elevated blood glucose level due to food intake - fasting blood glucose level) between the highest blood glucose level after intake and the fasting blood glucose level is lowered; the time needed for the level to decrease to the fasting blood glucose level after an elevation in blood glucose level is shortened; or the degree of elevation in postprandial blood glucose level represented by glycemic index (GI value) or the like is lowered, for example.

The expression "the highest value of an elevated blood glucose level due to food intake is lowered" unlimitedly refers to a case such that when a food for intake contains a yeast extract of yeast belonging to the genus *Saccharomyces,* the highest value of an elevated blood glucose level is lowered, by preferably 1 mg/dL or more, 5 mg/dL or more, 10 mg/dL or more, 20 mg/dL or more, 30 mg/dL, and 40 mg/dL or more, compared with a case where the food contains no such extract.

The expression "difference between the highest blood glucose level after intake and the fasting blood glucose level (the highest value of an elevated blood glucose level due to food intake - fasting blood glucose level) is lowered" unlimitedly refers to, a case such that when a food for intake contains a yeast extract of yeast belonging to the genus *Saccharomyces,* "the highest value of an elevated blood glucose level due to food intake - fasting blood glucose level" is lowered by preferably 1 mg/dL or more, 5 mg/dL or more, 10 mg/dL or more, 20 mg/dL or more, 30 mg/dL, and 40 mg/dL or more, compared with a case where the food contains no such extract.

The expression "after elevation in blood glucose level, the time needed for the level to decrease to the fasting blood glucose level is shortened" unlimitedly refers to a case such that when a food for intake contains a yeast extract of yeast belonging to the genus Saccharomyces, the time is shortened by preferably 1 minute or more, 5 minutes or more, 10 minutes or more, 20 minutes or more, 30 minutes or more, and 60 minutes or more, compared with a case where the food contains no such extract.

In the Description, the "effect of lowering a blood glucose level (s)" include as an aspect "effect of reducing a GI value(s)". The presence or the absence of the "effect of lowering a blood glucose level(s)" can be judged by determining the presence or the absence of the effect of reducing a GI value(s), for example.

The term "GI (Glycemic Index)" refers to the degree of absorption of carbohydrate contained in foods into blood, and is a numerical value indirectly representing the degree of an elevation in blood glucose level after intake of each food. Specifically, the GI is a measurement carried out on the amount of carbohydrate absorbed into blood for 2 hours after food intake. Specifically, the GI is calculated from the proportion of IAUC (incremental area under the curve) of a test diet (corresponding to 50 g of carbohydrate) when IAUC for 50 g of glucose is determined to be 100 (Jenkins, D.J., et al.,: Am. J. Clin. Nutr., 1981; 34: 362-366).

[Chemical Formula 1]

GI value = incremental area under the curve upon "food" intake/incremental area under the curve upon glucose intake × 100

Even with a low rate of elevation and a low peak, the IAUC of a food causing the blood glucose level to be kept at a high level for a long time period is increased and the GI value of the food is high. Moreover, the IAUC of a food that causes a rapid elevation in blood glucose level, and then secretion of a large amount of insulin, followed by a rapid decrease in the same is decreased, and the GI value of the food is low.

Generally, the term "low GI food" refers to a food with originally a low GI value, or, a food with a GI value decreased as a result of improvement in its production method or cooking method or addition of a substance having an effect of lowering blood glucose levels, and such a GI value is determined to be 55 or lower. In the description, the GI value of a low GI food is preferably 55 or lower, 50 or lower, 47 or lower, 45 or lower, and 42 or lower. The precise mechanism of a substance having an effect of lowering blood glucose levels is not particularly limited.

The term "food composition for reducing a GI value(s)" refers to, when a food for intake contains a yeast extract of yeast belonging to the genus *Saccharomyces,* a food composition having an effect of reducing (or suppressing a rise in) GI value, compared with a food containing no such extract. Furthermore, the term "food composition for reducing a GI value(s)" may refer to, when the food composition of the present invention is taken together with a food composition containing grain as a main raw material, a food composition having an effect of reducing (or suppressing a rise in) GI value, compared with a case where no food composition containing grain as a main raw material is taken together. The "compositions for reducing a GI value(s)" of the present invention include both food compositions: a food composition that contains a yeast extract of yeast belonging to the genus *Saccharomyces,* so as to reduce its own GI value; and, when taken with a food composition containing grain as a main raw material, a food composition having an effect of reducing the GI value of the food composition containing grain as a main raw material.

In the description, the expression "GI value is reduced" refers to, unlimitedly, a case such that when a food for intake contains a yeast extract of yeast belonging to the genus *Saccharomyces,* the GI value of the food composition is reduced, preferably by 10% or more, 20% or more, 30% or more, 35% or more, and 40% or more, compared with a food containing no such extract.

The type of the food composition of the present invention containing the yeast extract of yeast belonging to the genus *Saccharomyces* is not particularly limited. The type is preferably liquid, gel, paste, solid, powdery or granular, and is more preferably liquid. Examples thereof include modes such as Tsuyu (dipping sauce), Tare (sauce to be added), and sauce. The food composition of the present invention may be in a hot or cold state. The food composition of the present invention may be a food composition prepared by adding a yeast extract after cooking, or a food composition prepared by cooking (heating) the same containing a yeast extract. Moreover, the food composition of the present invention is preferably, but is not limited to, a food composition in which the yeast extract does not adversely affect the taste upon eating.

Subjects of the food of the present invention are unlimitedly humans. Such subjects may be healthy subjects, patients with diseases·symptoms, who need glucose control, and particularly patients with hyperglycemia (e.g., diabetes and drug-induced hyperglycemia). The subjects are preferably healthy subjects.

Method for enhancing the effect of a food composition for lowering blood glucose levels or method for reducing GI values

The present invention relates to a method for enhancing an effect of a food composition for lowering blood glucose levels, comprising incorporating a yeast extract of yeast belonging to the genus *Saccharomyces* into the food composition.

The present invention also relates to a method for reducing a GI value of a food composition, comprising incorporating a yeast extract of yeast belonging to the genus *Saccharomyces* into the food composition.

The terms "food composition", "yeast extract of yeast belonging to the genus *Saccharomyces",* "lowering a blood glucose level(s)", and "reducing a GI value(s)" are as described above for the food composition.

The expression "enhancing the effect of a food composition for lowering a blood glucose level(s)" refers to a case such that the food composition contains a yeast extract of yeast belonging to the genus *Saccharomyces,* so as to not only enhance the original effect (the effect that exists even when the food composition contains no yeast extract) of the food composition for lowering blood glucose levels, but also to impart an effect of lowering blood glucose levels to a food composition lacking an effect of lowering blood glucose levels.

Combination

The present invention further relates to a combination of the food composition of the present invention containing a yeast extract of yeast belonging to the genus *Saccharomyces* with a food composition containing grain as a main raw material. The combination of the present invention is used for particularly "lowering a blood glucose level(s)" or "reducing a GI value(s)", and thus is particularly useful. The present invention further relates to a set containing a food composition containing a yeast extract of yeast belonging to the genus *Saccharomyces,* and, a food composition containing grain as a main raw material. The set of the present invention can be used for enhancing an effect of lowering blood glucose levels, or, for reducing GI values.

(1) Food composition containing grain as a main raw material

The food composition containing grain as a main raw material is not particularly limited. Examples of grain include, but are not limited to, rice (including glutinous rice), wheat, barley, corn, rye, triticale, buckwheat, sorghum, millet, Japanese millet, and other cereals. The food composition containing grain as a main raw material may be a food composition prepared by cooking grain in their granular state, or a food composition prepared by processing these grain. An example of the former is rice, and an example of the latter is a processed food produced using a grain powder dough. Examples of a food composition made of processed grain include, unlimitedly, noodles, rice cake, bread, Okonomi-yaki (a thin, flat cake of unsweetened batter fried with various ingredients) (including Negiyaki (the pancake with green onion) and Hiroshima-yaki (the pancake with yakisoba noodles)), Tako-yaki (a cup type unsweeted butter fried with octopus), Monja-yaki (watery version of Okonomi-yaki), pizza, tortilla, dim sum, Chinese steamed buns, Korean pancakes, cereal foods or confectioneries (e.g., crepe).

Rice may be a mode of rice including cooked rice, fried rice, rice gruel, and paella, and a method for cooking rice is not particularly limited. Types and varieties of rice are also not limited.

Noodles are, unlimitedly, preferably selected from the group consisting of Udon noodle (thick Japanese noodles), Hiyamugi (thin Japanese noodles), Soumen (vermicellifine noodles), buckwheat noodles, pasta, Chinese noodles, and rice noodles. More preferably, noodles are Udon noodles. The present inventors have discovered that in the production process, noodles produced by a method comprising a mixing step by reduced-pressure mixing exert a lower GI value. This is because, but is not bound by logic, reduced-pressure mixing constructs a dense gluten network, and starch is gradually dissolved through the network after intake and then digested. A mixer for reduced-pressure mixing of a noodle dough is not particularly limited as long as a reduced-pressure environment can be established, and for example, a vacuum mixer or a decompression mixer can be used. The degree of vacuum employed for reduced-pressure mixing in the present invention, is preferably -400 mmHg or less, -500 mmHg or less, and -600 mmHg or less, when the normal atmospheric pressure is determined to be 0 mmHg, and the perfect vacuum is determined to be -760 mmHg. Moreover, the time for reduced-pressure mixing can be adequately selected by persons skilled in the art depending on dough conditions and the degree of vacuum. The time is preferably, but is not limited to, 5 minutes or longer. In a preferred aspect of the present invention, the food composition containing grain as a main raw material is a food composition produced by such a method comprising a mixing step by reduced-pressure mixing. In a preferred aspect of the present invention, the food composition containing grain as a main raw material is a noodle product, and is more preferably Udon noodle. In addition, such noodles may be in various modes of noodles, such as room-temperature noodles, refrigerated noodles, and frozen noodles.

Rice cake is a food produced by processing glutinous rice, and the type thereof is not particularly limited. Rice cake is broadly divided into two types: "Tsuki-mochi" produced by steaming granular rice and then pounding the rice with a mallet; and kneaded rice cake produced by adding hot water to grain powder, and then kneading and steaming the resultant, both of which are included as examples of the food composition containing grain as a main raw material of the present invention.

The type of bread is also not particularly limited. Examples of the bread include, in addition to general bread made of wheat as a material, rice bread made of rice as a material.

The types of Okonomi-yaki (including Negi-yaki and Hiroshima-yaki), Tako-yaki, Monja-yaki are also not particularly limited. These foods are made of wheat as a material, and ingredients contained therein are not limited.

The types of pizza and tortilla are also not particularly limited. Pizza is a food made by mixing and kneading wheat flour, water, salt, yeast, sugar, and a small quantity of olive oil, fermenting the dough, spreading the dough into a thin layer, placing topping ingredients thereon, and then baking the pizza dough in an oven, a dedicated oven, or the like. Tortilla is traditional thin-baked bread such as Mexican thin-baked bread made from ground corn. A similar food made from wheat flour is also referred to as tortilla.

Dim sum is a generic name of snacks of Chinese dishes. The type of dim sum in the present invention is not limited as long as it is dim sum made of grain as a main raw material. Examples of such dim sum include pan-fried dumplings (pot stickers), steamed meat dumplings, spring rolls, and soup dumplings.

Chinese steamed buns are sweet buns made by mixing and kneading wheat flour, water, sugar, yeast, baking powder and the like, fermenting the dough, wrapping ingredients with soft dough sheets, and then steaming the resultant. Examples of Chinese steamed buns include buns with meat filling and buns with bean-jam filling depending on the types of ingredients, but the types are not limited in the present invention.

Korean pancake is made by mixing wheat flour, rice flour, water, eggs and appropriate ingredients (e.g,. onion, chive, carrot, and green onion) to prepare a dough, and baking the dough by frying-like baking using a frying pan with a more than sufficient amount of oil. This is also referred to as Korean-style Okonomi-yaki.

Cereal foods are convenient foods processed by cooking with heat, such as by grinding grain such as corn, oats, wheat, barley, or rice into thin fragments (flakes), a puff form (expanded), mixing, shaping into sheets, and then crushing the sheets, so that it can be easily eaten, for example, and then shaping them into forms appropriate for long-term storage. In the present invention, the type of such a cereal food is not particularly limited, and examples thereof include cornflakes and oatmeal.

The types of confectioneries are also not particularly limited. Examples of the types include Japanese confectioneries such as sweet buns and European confectionaries such as cake, crepe, and galette.

Such food composition containing grain as a main raw material is rich in carbohydrate, and is mostly served as the main dish of a diet.

In the combination of the present invention, the type of a food composition containing a yeast extract of yeast belonging to the genus *Saccharomyces* is not particularly limited. Preferably, the type is liquid, gel, paste, solid, powdery or granular. More preferably, the type is liquid. Examples thereof include modes such as Tsuyu, Tare, and sauce. The food composition may be in a hot state or cold state.

When the food composition containing grain as a main raw material, such as noodles contain a yeast extract, the physical properties (e.g., smoothness, viscosity, and elasticity) are altered, and the taste and the texture may be deteriorated. Through combination of the food composition containing grain as a main raw material with, for example, a liquid food composition containing a yeast extract of the present invention, problems such as deteriorated taste and texture can be addressed.

For example, when the food composition containing grain as a main raw material is a noodle, as a food composition containing a yeast extract, liquid Tsuyu (e.g., dipping sauce for Udon noodle), Tare, soup, Shiru (soup or broth) or the like can be provided. The type of noodles may be a "Tsuke-men type (this type of noodles is dipped in a dipping sauce to eat)" in which Tsuyu and noodles are separately provided in a pack, or "Kake-men type (this type of noodles is served with soup to eat)" in which soup (Shiru) is spread over the noodles.

Further examples of a combination of the food composition containing grain as a main raw material and a food composition containing a yeast extract include, but are not particularly limited to, as long as they are usually eaten in combination, Ochazuke (boiled rice with tea and Dashi-jiru), rice and curry of curry rice, cooked rice and miso soup, Okonomi-yaki and sauce, cake and black tea or coffee.

The expression "...taken (eaten) in combination" means, when the food composition containing grain as a main raw material is a noodle, not only a case such that the food composition and a food composition containing a yeast extract are brought into the mouth completely simultaneously (for example, Udon noodle and Men-tsuyu, cooked rice and Dashi-jiru of Ochazuke, and Okonomi-yaki and sauce), but also a case such that food compositions are taken into the body substantially simultaneously in a single diet. In the present invention, both food compositions are eaten in combination, so that the active ingredients of the yeast extract are absorbed *in vivo,* an elevation in blood glucose level due to digestion and absorption of the food composition containing grain as a main raw material can be suppressed, and/or, an effect of accelerating a decrease in blood glucose level can be obtained. When a food composition containing a yeast extract is liquid, the active ingredients thereof are rapidly absorbed, suppression of an elevation in blood glucose level due to digestion and absorption of the food composition containing grain as a main raw material, which is eaten simultaneously therewith/acceleration of a decrease in blood glucose level can be achieved more effectively, and thus this is more preferable.

(2) Resistant starch

In an aspect of the present invention, the food composition containing grain as a main raw material contains a resistant starch.

"Resistant starch" (RS) is a generic name of starch and degradation products having resistance to the digestive effects of digestive enzymes, and are not digested and absorbed in the small intestine of a healthy individual. The resistant starch is also referred to as indigestible starch or tolerance starch. RS is insoluble in water, and thus water-soluble indigestible dextrin is not included in examples of RS. RS is classified into the following four types depending on differences in mechanisms for the indigestible nature.

RS 1: Digestive enzymes are inaccessible to this type of RS since it is surrounded by hard tissue, such as cereals.

RS 2: This type of RS has starch particles that are not easily digested, such as not sufficiently heated, ungelatinized starches and extremely amylose-rich starches.

RS 3: This type of RS has an altered structure that is not easily digested as a result of recrystallization of some starches through processes of cooling and storage after heating and gelatinization, such as cooled cooked rice or Harusame.

RS 4: This type of RS is a processed starch formed via chemical modification thereof to a high degree, so as to be resistant to the effects of digestive enzymes.

In the present invention, the type of RS and the method for producing RS are not particularly limited, but RS is preferably separated·purified from corn, rice or the like having a high amylose content.

The RS in the present invention preferably has high heat stability. RS known as an example of RS having high heat stability is prepared by subjecting high-amylose corn starch to acid treatment, so as to have a molecular weight peak of $6\times10^3$ or more and $4\times10^4$ or less. Specifically, this RS is prepared by performing hydrolysis with acid to reduce the starch molecular weight to one-tenth that of the starch, to achieve dense spatial arrangement of molecules, to increase the crystallinity of amylose molecules, and thus to improve the heat stability and resistance to digestion. Note that such high-amylose corn starch is a corn starch having an increased amylose content through breeding, and preferably has an amylose content of 40% or more. Acid treatment can be performed by mixing inorganic acid such as hydrochloric acid, sulfuric acid, or nitric acid with starch and purified water.

Moreover, as a RS-rich starch to be used in the present invention, a RS-rich starch that can be measured by the AOAC official method 2002. 02 that is an assay procedure according to conditions for *in vivo* digestion of starch is preferred.

An example of such RS-rich starch that satisfies the conditions is RS disclosed in Japanese Patent Laid-Open No. 2011-84674 (JP Patent No. 4482611).

Specifically, as a preferred aspect of the present invention, the food composition containing grain as a main raw material contains as RS a RS-rich starch that satisfies the following conditions (a), (b), (c) and (d):

(a) The resistant starch content is 60% or more as measured by the resistant starch measurement method in accordance with the AOAC official method 2002.02.

(b) The molecular weight peak is $6 \times 10^3$ or more and $4 \times 10^4$ or less.

(c) The molecular weight dispersion degree is 1.5 or more and 6.0 or less.

(d) The enthalpy of gelatinization at 50°C to 130°C is 10 J/g or less as measured by differential scanning calorimetry.

Such RS-rich starch may be RS that is obtained by subjecting a amylose-rich starch having a high amylose content of 40% or more as a raw material to acid treatment in an aqueous solution of inorganic acid. The reaction conditions of the acid treatment satisfy, unlimitedly, the following Chemical formulae (1) and (2).

[Chemical Formula 2]

$$(5.54\times(4.20)(T-40)/10)(-0.879)\leq C<-0.000016\times T3+0.00068\times T2-0.028\times T+4.3 \quad (1)$$

$$13.0\times C(-1.14)\times(1/4.2)(T-40)/10\leq t\leq 180\times C(-1.58)\times(1/4.2)(T-40)/10 \quad (2)$$

(In the above Chemical formulae (1) and (2), T: reaction temperature (°C), C: normality (N) of inorganic acid in the aqueous solution of inorganic acid, and t: reaction time (hour).)

An example of a RS-rich starch that satisfies the above conditions, is AMYLOFIBER (Registered Trademark) SH (J-OIL MILLS INC.). AMYLOFIBER (Registered Trademark) SH contains RS accounting for about 60%, as measured by the resistant starch measurement method in accordance with the AOAC official method 2002. 02. Moreover, the molecules are crystalized to such an extent that the molecular weight peak is as low as $1.2\times10^4$, so that the resulting product has high heat stability. Hence, the product can be preferably used as a starch having a high RS content of the present invention.

Preferably, the food composition containing grain as a main raw material contains RS in an amount of 0.6 wt% or more and less than 6 wt%, preferably 1 wt% or more and 5 wt% or less, with respect to the total amount of powder raw materials (the total amount of grain as the main raw material, other powders or flours, and an RS-containing raw material). For example, in the case of Udon noodle, the total amount of powder raw materials represents the total amount of wheat flour and an RS-containing starch. The powder raw materials in this case may further contain as other powders or flours a processing aid and the like. Furthermore, RS-containing Udon noodle is preferably, but is not limited to, RS-containing Udon noodle produced by a method comprising a reduced-pressure step.

In the Examples of the present invention, Udon noodle was prepared using AMYLOFIBER (Registered Trademark) SH in an amount of about 5 wt% in powder raw materials. AMYLOFIBER (Registered Trademark) SH contains RS accounting for about 60% thereof. Therefore, the RS content is about 3 wt% in the powder raw materials. The amount of AMYLOFIBER (Registered Trademark) SH is 1 wt% or more and less than 10 wt%, and is preferably 1 wt% or more and 8 wt% or less with respect to the powder raw materials.

Method for enhancing the effect of food composition for lowering blood glucose level or method for reducing GI value

The present invention further relates to a method for enhancing an effect of lowering blood glucose levels of both of the food composition of the present invention containing a yeast extract of yeast belonging to the genus *Saccharomyces* and a food composition containing grain as a main raw material, comprising combining the food compositions.

The present invention further relates to a method for reducing GI values of both of the food composition of the present invention containing a yeast extract of yeast belonging to the genus *Saccharomyces* and a food composition containing grain as a main raw material, comprising combining the food compositions.

The expressions and terms "food composition containing a yeast extract of yeast belonging to the genus *Saccharomyces*", "food composition containing grain as a main raw material", "combination", "lowering a blood glucose level(s)", "enhancing the effect of a food composition(s) for lowering a blood glucose level(s)", and "reducing a GI value(s)" are as defined above for food compositions and combinations.

Use of combination

The present invention further relates to the use of a combination of the food composition of the present invention containing a yeast extract of yeast belonging to the genus *Saccharomyces* with the food composition containing grain as a main raw material for the method for enhancing the effect of the food compositions for lowering blood glucose levels, or, the use of a combination of the food composition of the present invention containing the yeast extract of yeast belonging to the genus *Saccharomyces* with a food composition containing grain as a main raw material for the method for reducing GI values.

The expressions and terms "food composition containing a yeast extract of yeast belonging to the genus *Saccharomyces*", "food composition containing grain as a main raw material", "combination", "lowering a blood glucose level(s)", "enhancing the effect of food compositions for lowering a blood glucose level(s)", and "reducing a GI value(s)" are as described above for food compositions and combinations.

Noodles for lowering blood glucose level

The present invention further relates to noodles for lowering blood glucose levels. The noodles of the present invention contain a resistant starch, are produced by a method comprising a mixing step by reduced-pressure mixing, and have an application for lowering blood glucose levels. The expression "lowering a blood glucose level(s)" is as described above for food compositions and combinations. The noodles of the present invention are low GI foods having reduced GI values, and exert an effect of lowering blood glucose levels (including an effect of reducing the GI value) when eaten.

The expressions and terms "noodles", "resistant starch", and "mixing step by reduced-pressure mixing" are as described above.

Method for reducing the GI value of noodles

The present invention further relates to a method for reducing the GI value of noodles. The method of the present invention comprises blending noodles with a resistant starch, and producing the noodles by a method comprising a mixing step by reduced-pressure mixing.

The expressions and terms "noodles", "reducing a GI value(s)", "resistant starch", and "mixing step by reduced-pressure mixing" are as described above.

EXAMPLES

Hereinafter, the present invention will be described more specifically on the basis of Examples, but, the present invention is not limited to them. Persons skilled in the art can easily add modifications and/or changes to the present invention based on the content of the Description, and the modifications and changes are included in the technical scope of the present invention.

Materials and methods

The following materials and methods are employed unless otherwise specified in the Examples of the Description.

[Preparation of vacuum Udon noodle]

Vacuum Udon noodle was prepared as follows.

(1) Vacuum Udon noodle

A dough obtained by adding 500 g of salt water to 1000 g of wheat flour, and then performing 10 minutes of reduced-pressure mixing (while keeping the degree of vacuum at - 500 mmHg or lower) of the mixture using a horizontal-type vacuum mixer (Sanuki Menki Co.,Ltd.) for noodle making was rolled, combined, and then matured by conventional methods. Subsequently, the dough was rolled by a conventional method so as to obtain raw Udon noodle having a noodle width of 3.8 mm and a noodle thickness of 3.2 mm. The raw Udon noodle was then cooked by boiling in a sufficient amount of boiling hot water for about 10 minutes, immediately washed with water, and then cooled in cold water, so as to obtain cooked Udon noodle (boiled Udon noodle). Note that, in the following Examples, the term "vacuum Udon noodle" indicates Udon noodle obtained by the above steps unless otherwise specified.

(2) RS vacuum Udon noodle

To 950 g of wheat flour, 50 g of AMYLOFIBER (Registered Trademark) SH (J-OIL MILLS INC., high-amylose corn starch degradation product containing a resistant starch (RS) accounting for about 60% (as measured using the AOAC official method 2002. 02)) and 500 g of salt water (AMYLOFIBER (Registered Trademark) SH accounted for 5 wt% of powder raw materials, and thus RS accounted for 3 wt% of the powder raw materials) were added. The mixture was subjected to 10 minutes of reduced-pressure mixing (while keeping the degree of vacuum at -500 mmHg or lower) using a horizontal-type vacuum mixer for noodle making (Sanuki Menki Co.,Ltd.), and then the thus obtained dough was rolled, combined, and then matured by conventional methods. Subsequently, the dough was rolled by a conventional method, so as to obtain raw Udon noodle having a noodle width of 3.8 mm and a noodle thickness of 3.2 mm. The raw Udon noodle was then cooked by boiling in a sufficient amount of boiling hot water for about 10 minutes, immediately washed with water, and then cooled in cold water, so as to obtain cooked Udon noodle (boiled Udon noodle). In addition, in the following Examples, the term "RS vacuum Udon noodle" indicates Udon noodle obtained by the above steps unless otherwise specified.

[Method for conducting test for measuring blood glucose level]

(1) Blood glucose level was measured by performing fingertip blood collection using Medisafe FIT (TERUMO Corporation).
(2) Subjects were instructed to fast by skipping breakfast and then the test was started at 8AM to 9AM.
(3) Fingertip blood collection was performed before intake of standard diet·test diet, 15 minutes, 30 minutes, 45 minutes, 60 minutes, 90 minutes, and 120 minutes after intake.
(4) A subject having a fasting blood glucose level before intake ranging from 70 to 110 underwent the test.
(5) An experimental diet and a test diet were served once a day, and the washout period was at least one day after the previous blood collection.

[Method for calculating GI value]

(1) Blood collection after challenge with Standard diet (50 g of Trelan (Registered Trademark) G (AY Pharmaceutical Co., Ltd.): 50 g of carbohydrate) was performed twice.
(2) If a difference in incremental area under the curve (hereinafter, referred to as "IAUC".) was within 25%, the results were averaged and determined to be a reference value. If a difference in the same was 25% or more, a third challenge test was conducted, the results, each of which was within 25%, were averaged and the average was determined to be a reference value.

(3) A challenge test with a test diet prepared to contain 50 g of carbohydrate was conducted, and then IAUC was found.

(4) The proportion of the thus calculated IAUC of the test diet in that of the standard diet was calculated, and the obtained value was determined to be the GI value of each subject. The GI values of subjects were averaged, values greater than twice the standard deviation were excluded, the GI values were averaged again, and thus the resulting value was determined to be the GI value of the test diet.

$$\text{GI value} = ((\text{IAUC of test diet})/(\text{IAUC of standard diet})) \times 100$$

Example 1 Effect of reducing GI value through combination of Udon noodle with Tsuyu supplemented with yeast extract

In this Example, the effect of reducing GI values through combination of Udon noodle with Tsuyu supplemented with a yeast extract was examined.

(1) Male and female adults were randomly chosen as panelists.

(2) Vacuum Udon noodle or RS vacuum Udon noodle produced as described above were combined with Men-tsuyu (noodle dipping sauce), and then each panelist was instructed to eat the combination as cold Udon noodle.

Udon noodle: cold Udon noodle (167 g of Udon noodle (the amount of carbohydrate: 48 g));
Men-tsuyu supplemented with yeast extract (12.3 g): Oigatsuo-tsuyu (bonito broth supplemented with additional pieces of dried bonito) (Registered Trademark) (Mizkan Holdings Co., Ltd., the amount of carbohydrate: 2 g) supplemented with 0.025 g of a yeast extract, "Yeast Extract 21-NYP" (Fuji Foods Corporation. Hereinafter, referred to as "NYP".) (yeast extract content: about 0.2 wt% of Men-tsuyu + yeast extract), or
Men-tsuyu 12.3 g: Oigatsuo-tsuyu (Registered Trademark) (Mizkan Holdings Co., Ltd., amount of carbohydrate: 2 g)
As a Comparative Example of Udon noodle, in the above step of producing vacuum Udon noodle, 167 g of Udon noodle (Hereinafter, referred to as "atmospheric Udon noodle".) produced not by mixing under reduced pressure, but by mixing under atmospheric pressure and the above Men-tsuyu were used. Combinations listed in Table 1 were subjected to measurement of blood glucose levels according to the above method for measuring blood glucose levels, and then confirmed for the effects.

[Table 1]

| No. | No. 1 | No. 2 | No. 3 | No. 4 | Comparative Example |
|---|---|---|---|---|---|
| Udon noodle | Vacuum Udon noodle | RS Vacuum Udon noodle | Vacuum Udon noodle | RS Vacuum Udon noodle | Atmospheric Udon noodle |
| Yeast extract added to Tsuyu | No supplementation | No supplementation | NYP | NYP | No supplementation |
| n (number) | 7 | 7 | 9 | 8 | 5 |
| GI value | 55.3 | 53.2 | 42.6[a] | 41.7[a] | 65.9 |
| Standard deviation | 19.8 | 10.0 | 19.0 | 9.8 | 13.8 |
| Figure number | Fig. 1 | Fig. 2 | Fig. 3 | Fig. 4 | Fig. 5 |
| a: indicates that a significant difference was confirmed at a significance level of 5% with respect to Comparative Example. | | | | | |

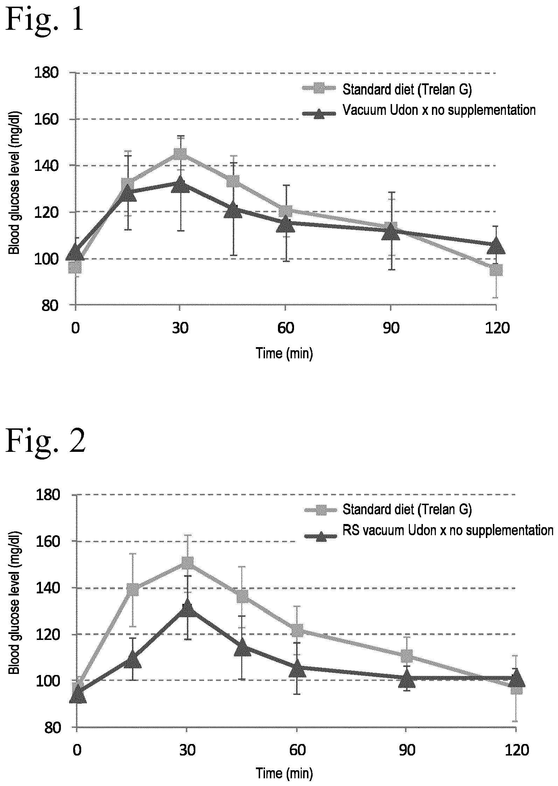

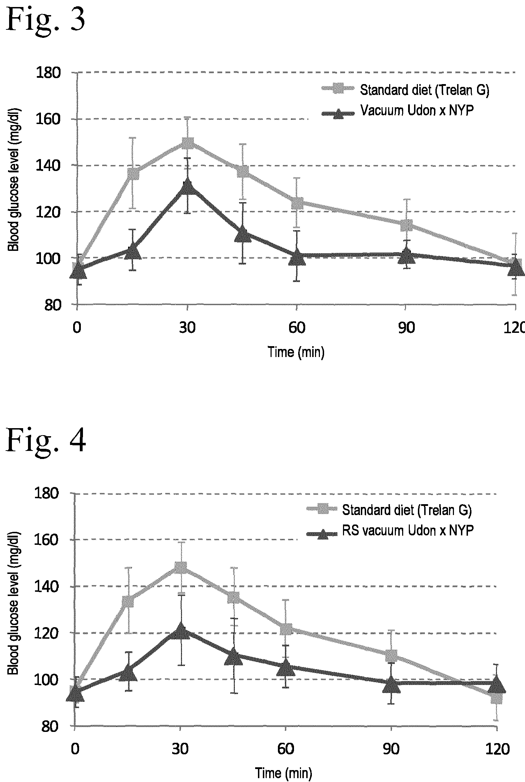

As shown in Table 1 and Fig. 1 to Fig. 5, vacuum Udon noodle was found to have a GI value lower than that of atmospheric Udon noodle (comparison between No. 1 and Comparative Example). Furthermore, it was found that the addition of NYP-containing Tsuyu (dipping sauce) to vacuum Udon noodle enhanced the effect (No. 3). Moreover, the results of standard deviation demonstrate that simultaneous eating of vacuum Udon noodle supplemented with RS and NYP-containing Tsuyu allows stable exertion of the significant effect of reducing GI values (No. 4).

Example 2 Evaluation of texture of low GI Udon noodle

In this Example, the texture of low GI Udon noodle made of the combinations of the present invention was evaluated.

(1) Production of vacuum Udon noodle

Vacuum Udon noodle was produced by mixing with compositions listed in Table 2 below according to the above method.

[Table 2]

|  | RS-rich vacuum Udon noodle | Indigestible dextrin-rich vacuum Udon noodle | NYP-containing vacuum Udon noodle |
|---|---|---|---|
| Wheat flour | 700g | 800g | 980g |
| Content | RS 300g | Indigestible dextrin (Fibersol-2 (Matsutani Chemical Industry Co., Ltd.)) 200g | NYP 20g |
| Total | 1000g | 1000g | 1000g |

(2) Three types of Udon noodle obtained in (1)(RS-rich vacuum Udon noodle, indigestible dextrin-rich vacuum Udon noodle, NYP-containing vacuum Udon noodle) and vacuum Udon noodle and RS vacuum Udon noodle produced in Example 1 (total 5 types of Udon noodle) were combined with Men-tsuyu as in Table 3, and each panelist was instructed to eat each combination of Udon noodle and Men-tsuyu, as cold Udon noodle.

Udon noodle: cold Udon noodle (167 g of Udon noodle (amount of carbohydrate: 48 g))

Men-tsuyu supplemented with yeast extract (12.3 g): Oigatsuo-tsuyu (Registered Trademark) (Mizkan Holdings Co., Ltd., the amount of carbohydrate: 2 g) + NYP 0.025g, or

Men-tsuyu (12.3 g): Oigatsuo-tsuyu (Registered Trademark) (Mizkan Holdings Co., Ltd., the amount of carbohydrate: 2 g)

Each combination was subjected to measurement of the blood glucose level according to the above method for measuring blood glucose levels, and then confirmed for the effect.

(3) Ten well-trained panelists made sensory evaluations for the physical properties; that is, evaluation items of each Udon noodle noodle product (smoothness, viscosity, and elasticity) based on a 5-point evaluation system ranging from 1 (poor) to 5(good), and the scores marked by each panelist were averaged. Moreover, the average of the thus obtained score of viscosity and score of elasticity was designated as viscoelasticity. The results of (2) and (3) are shown in Table 3. Furthermore, viscoelasticity was plotted along the vertical axis and smoothness was plotted along the horizontal axis (Fig. 6)

[Table 3]

| No. | No. 1 | No. 2 | No. 3 | No. 4 | No. 5 | Comparative Example |
|---|---|---|---|---|---|---|
| Udon noodle | RS-rich vacuum Udon noodle | Indigestible dextrin-rich vacuum Udon noodle | NYP-containing vacuum Udon noodle | Vacuum Udon noodle | RS Vacuum Udon noodle | Vacuum Udon noodle |
| Men-tsuyu | No supplementation with yeast extract | No supplementation with yeast extract | No supplementation with yeast extract | Supplementation with NYP | Supplementation with NYP | No supplementation with yeast extract |
| GI value | 32.3 | 56.7 | 44.3 | 42.6 | 41.7 | 55.3 |
| Smoothness of noodles | 1.5 | 4 | 4.5 | 4.5 | 4 | 4.5 |
| Viscosity of noodles | 3.3 | 2.3 | 4.3 | 4.2 | 4.3 | 4.2 |
| Elasticity of noodles | 2.8 | 2 | 4 | 4 | 4.5 | 4 |
| Viscoelasticity of noodles (=(viscosity + elasticity)/2) | 3.05 | 2.15 | 4.15 | 4.1 | 4.35 | 4.1 |
| Comment | Powdery and dry texture | No chewiness of noodles felt | Noodles were discolored brown. Harsh taste and yeast extract odors were felt when eaten. | Good texture. | Good texture. | Good texture. |

As shown in Table 3 and Fig. 6, RS-rich vacuum Udon noodle (No. 1) was found to have a low GI value but have powdery and dry texture, indigestible dextrin-rich vacuum Udon noodle (No. 2) was found to have a high GI value and lacks chewy texture, and NYP-containing vacuum Udon noodle (No. 3) was found to have a somewhat reduced GI value and good texture, but be problematic in color and taste. In contrast, a combination of RS-containing vacuum Udon noodle with Men-tsuyu supplemented with NYP (No. 5) was found to be able to simultaneously realize low GI, good texture, and good taste.

Example 3 Type and effect of reducing GI value of Yeast extract

In this Example, various types of yeast extract were examined for the effect of reducing GI values.

(1) Six to nine male and female adults were randomly chosen as panelists.
(2) RS vacuum Udon noodle prepared to contain about 50 g of carbohydrate and Men-tsuyu were combined, and each panelist was instructed to eat the combination as cold Udon noodle.

Udon noodle: RS vacuum Udon noodle (167 g of Udon noodle (the amount of carbohydrate: 48 g))
Men-tsuyu supplemented with yeast extract (12.3 g): Oigatsuo-tsuyu (Registered Trademark) (Mizkan Holdings Co., Ltd., the amount of carbohydrate: 2 g) + various types of yeast extract (0.025 g) listed in Table 4, or
Men-tsuyu (12.3 g): Oigatsuo-tsuyu (Registered Trademark) (Mizkan Holdings Co., Ltd., the amount of carbohydrate: 2 g)
The blood glucose level was measured before intake of a test diet, and at 15 minutes, 30 minutes, 45 minutes, 60 minutes, 90 minutes, and 120 minutes after intake.

[Table 4]

| No. | No. 1 | No. 2 | No. 3 | No. 4 | No. 5 | No. 6 | No. 7 |
|---|---|---|---|---|---|---|---|
| Yeast extract | No supplementation | NYP (Fuji Foods Corporation) | Vertex IG20 (Fuji Foods Corporation) | Sp-d (YEASTOCK) | Ajilex LK (Kohjin co., Ltd.) | Aromild (Kohjin co., Ltd.) | Yeast Extract 21-TF (Fuji Foods Corporation) |
| Type of yeast | - | Saccharomyces. cerevisiae | Saccharomyces. cerevisiae | Saccharomyces. cerevisiae | Candida. utilis | Candida. utilis | Candida. utilis |
| Characteristics of yeast extract | - | High amino acid type | High nucleic acid type | High amino acid type (particularly, glutamic acid) | Nucleic acid-and-amino acid-balanced type | High nucleic acid type | High amino acid type |
| n (number) | 7 | 8 | 9 | 6 | 6 | 8 | 6 |
| GIvalue | 53.2 | 41.7* | 41.6** | 33.6*** | 50.6 | 53.2 | 52.2 |
| Standard deviation | 10.0 | 9.8 | 8.2 | 9.6 | 25.1 | 22.6 | 11.4 |
| Figure | Fig. 2 | Fig. 4 | Fig. 7 | Fig. 8 | Fig. 9 | Fig. 10 | Fig. 11 |

*: indicates that a significant difference was confirmed at a significance level of 10% with respect to No. 1.
**: indicates that a significant difference was confirmed at a significance level of 5% with respect to No. 1.
***: indicates that a significant difference was confirmed at a significance level of 1% with respect to No. 1.

EP 3 443 849 A1

As a result, the use of the yeast extract extracted from yeast belonging to the genus *Saccharomyces* was found to enable stable suppression of elevations in blood glucose level. Moreover, the yeast extract extracted from Torula *yeast* (*Candida utilis*) was observed to have no such effect.

- Ajilex LK: Torula *yeast* (*Candida utilis*)-derived yeast extract rich in inosinic acid, guanylic acid, and other nucleic acids and peptides.

- Aromild: the yeast extract produced by subjecting an extract extracted from Torula *yeast* (*Candida utilis*) by hot water extraction to enzyme treatment using nuclease and the like, so as to degrade RNA components contained therein into nucleotides.

Example 4 Fractionation test for ingredients contained in yeast extract

In this Example, a fractionation test was conducted for ingredients contained in yeast extract (NYP). Specifically, the fractionation solutions of ingredients contained in yeast extracts were prepared according to the following procedures.

(A) Procedures

(1) NYP was dissolved in water to prepare a 10% aqueous solution.
(2) Fractionation was performed using a pencil-type module (Asahi Kasei Chemicals) having a fractionation molecular weight of 3000 (for 24 hours while cooling the container with ice).
(3) A sample that had remained unpermeated was designated as fractionation solution 1.
(4) A dialysis tube (Spectra/Por (Funakoshi Co., Ltd.)) having a fractionation molecular weight ranging from 500 to 1000 was filled with a fraction that had permeated, followed by dialysis with agitation in distilled water (4°C, 24 hours, distilled water 1 L $\times$ 2 times of exchange).
(5) The sample within the dialysis tube was designated as fractionation solution 2.
(6) A portion on the distilled water side was vacuum-concentrated in such a manner that the amount thereof was equivalent to that before dialysis.
(7) A dialysis tube having a fractionation molecular weight ranging from 100 to 500 (Spectra/Por, Funakoshi Co., Ltd.) was filled with (6), followed by dialysis with agitation in distilled water (4°C, 24 hours, distilled water 1 L $\times$ two times of exchange).
(8) The sample within the dialysis tube was designated as fractionation solution 3.
(9) A portion on the distilled water side was vacuum-concentrated in such a manner that the amount thereof was equivalent to that before dialysis, and was designated as fractionation solution 4.
(10) Each fractionation solution was concentrated using an evaporator, and then pulverized by freeze drying using a freeze dryer.

(B) Details about fractionation

The molecular weight of the ingredient contained in each fractionation solution is shown in the following Table.

[Table 5]

| Fraction | Molecular weight |
|---|---|
| Fraction 1 | Molecular weight >3000 |
| Fraction 2 | 500 - 1000 <Molecular weight <3000 |
| Fraction 3 | 100 - 500 <Molecular weight <500 - 1000 |
| Fraction 4 | Molecular weight <100 - 500 |

(C) Result of evaluating decrease in GI of each fractionation solution

The GI value of each fractionation solution of NYP was measured by the above method. Furthermore, for comparison, the GI values of free branched-chain amino acids (BCAA) in the same amounts (Table 6) as those of NYP, the types and the amounts of nucleic acids (Table 7) contained in a nucleic acid-rich yeast extract, Aromild (Kohjin co.,

Ltd.), and a control were measured by the above method.

[Table 6]

| BCAA | | |
|---|---|---|
| | Amount of BCAA added | Maker |
| L-Leu | 1.67 mg | Tokyo Chemical Industry Co., Ltd. |
| L-Ile | 1.19 mg | Tokyo Chemical Industry Co., Ltd. |
| L-Val | 2.13 mg | Wako Pure Chemical Industries, Ltd. |

[Table 7]

| Nucleic acid | | | |
|---|---|---|---|
| | Amount added (mg) | Maker | (Nucleic acid ratio) |
| 5'-IMP·2Na·7H2O | 2.7 | Sigma-Aldrich | 10.73 |
| 5'-GMP·2Na·7H2O | 2.5 | Sigma-Aldrich | 9.81 |
| 5'-AMP·2Na·7H2O | 0.02 | Sigma-Aldrich | 0.09 |
| 5'-CMP·2Na·7H2O | 1.7 | Sigma-Aldrich | 6.78 |
| 5'-UMP·2Na·7H2O | 1.9 | Sigma-Aldrich | 7.66 |

[Table 8]

| | Control | Fraction 1 | Fraction 2 | Fraction 3 | Fraction 4 | BCAA | Nucleic acid |
|---|---|---|---|---|---|---|---|
| Yeast extract | No supplementation | NYP Molecular weight >3000 | NYP 500 - 1000 <Molecular weight <3000 | NYP 100 - 500 <Molecular weight <500 - 1000 | NYP Molecular weight <100 - 500 | BCAA | Nucleic acid |
| n (number) | 7 | 8 | 7 | 5 | 7 | 9 | 8 |
| GI value | 53.2 | 57.8 | 40.2 | 59.2 | 63.4 | 56.8 | 51.8 |
| Standard deviation | 24.8 | 23.6 | 22.2 | 16.4 | 21.4 | 17.8 | 20.2 |
| Figure | | Fig. 12 | Fig. 13 | Fig. 14 | Fig. 15 | Fig. 16 | Fig. 17 |

GI value was significantly reduced only in Fraction 2 (500 to 1000 < molecular weight < 3000). Moreover, no significant reduction in GI value was observed for BCAA or nucleic acid. The above results demonstrated that a substance having the effect of reducing GI values was present in the fraction (500 to 1000 < molecular weight < 3000) when the above fractionation method was employed.

(D) HPLC analysis of the molecular weight distributions of fractionation solutions

The molecular weight distributions of the substances contained in NYP and each of the above fractionation solutions were analyzed by HPLC. D-7000 Series (Hitachi, Ltd.) was used as a HPLC system, GS-320 HQ (Asahipak) coupled to GS-320 HQ (Asahipak) (Showa Denko K.K.) was used as a column, and then measurement was performed under the measurement conditions shown in Table 9. The thus obtained percentages of molecular weight distributions are shown in Table 10.

[Table 9]

| Eluent | H$_2$O |
|---|---|
| Flow rate of eluent | 0.6ml / min |
| Column oven | 30°C |
| Concentration of sample | 0.5% |
| Injection amount of sample | 20 μl |

[Table 10]

| | ≥10000 | 3000 - 10000 | 500 - 3000 | ≤500 |
|---|---|---|---|---|
| NYP | 18.0% | 5.5% | 21.7% | 54.9% |
| Fraction 1 (Molecular weight >3000) | 35.3% | 5.0% | 11.1% | 48.5% |
| Fraction 2 (500 - 1000 <Molecular weight <3000) | 3.5% | 3.8% | 20.3% | 72.4% |
| Fraction 3 (100 - 500 <Molecular weight <500 - 1000) | 0.4% | 1.4% | 12.5% | 85.7% |
| Fraction 4 (Molecular weight <100 - 500) | 0.5% | 1.0% | 6.5% | 92.1% |

It was confirmed by the above results that since the proportion of a substance with a molecular weight ranging from 500 to 3000 in NYP was higher than those of Fraction 1, Fraction 3, and Fraction 4, and equivalent to that of Fraction 2, an ingredient that reduces the GI value was contained in Fraction 2.

Example 5 Effect of reducing GI value exerted by combination of Udon noodle and Tsuyu supplemented with yeast extract

In this Example, a combination of Udon noodle and Tsuyu supplemented with a yeast extract was examined for the effect of reducing GI values. The following test was conducted under non disclosure agreement at an external contract testing laboratory (CPCC Co., Ltd.).

(1) Subjects of this test were twenty male and female healthy subjects who were 20-year-old or older and 50-year-old or younger, had BMI of 30 Kg/m$^2$ or lower. Finally, the GI values of 12 subjects were employed.

(2) RS vacuum Udon noodle produced as described above and Men-tsuyu were combined, and each panelist was instructed to eat the combination as cold Udon noodle.
Udon noodle: cold Udon noodle (167 g of Udon noodle (the amount of carbohydrate: 48 g));
Men-tsuyu supplemented with yeast extract (12.3 g): To Oigatsuo-tsuyu (Registered Trademark) (Mizkan Holdings Co., Ltd., the amount of carbohydrate: 2 g), 0.025 g of a yeast extract "Yeast Extract 21-NYP" (Fuji Foods Corporation. Hereinafter, referred to as "NYP".) was added (yeast extract content: about 0.2 wt% of Men-tsuyu + yeast extract), or Men-tsuyu (12.3 g): Oigatsuo-tsuyu (Registered Trademark) (Mizkan Holdings Co., Ltd., the amount of carbohydrate: 2 g)

Test diet 1: RS Udon noodle + NYP
Test diet 2: RS Udon noodle + Men-tsuyu with no supplementation

A challenge test using a referential glucose solution (Trelan G: 50 g) was conducted twice on subjects excluding those within disease area and those with impaired glucose tolerance. Blood glucose levels were measured according to the above method for measuring blood glucose levels and then the effect was confirmed.

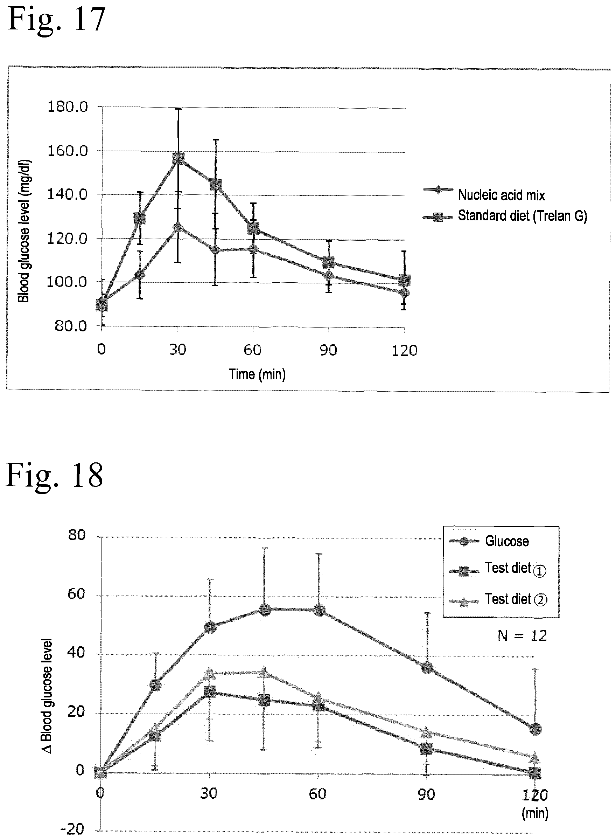

The results are shown in Fig. 18. In the results in Fig. 18, test diet 1(the present invention) was found to have a GI value of 39.8, and test diet 2(Comparative Example) was found to have a GI value of 52.4.

Example 6 Effect of reducing GI value exerted by combination of Okonomi-yaki and sauce supplemented with yeast extract

In this Example, the effect of reducing GI values exerted by the combination of Okonomi-yaki and a sauce supplemented with a yeast extract was examined.

(1) Eight to ten male and female adults were randomly chosen as panelists.
(2) The following Okonomi-yaki was combined with a sauce supplemented with the yeast extract or the sauce, and each panelist was instructed to eat the combination.

Okonomi-yaki: "Gottsu Umai Okonomi-yaki" (Tablemark Co., Ltd. (341 g) (the amount of carbohydrate: 38.5 g); Sauce supplemented with yeast extract (30 g): To the sauce (the amount of carbohydrate: 11.5 g) provided together with the above "Gottsu Umai Okonomi-yaki", 0.5g of yeast extract NYP (yeast extract NYP content: about 1.6 wt% of sauce + yeast extract NYP) was added, or
Sauce (30 g): The sauce (the amount of carbohydrate: 11.5 g) provided together with the above "Gottsu Umai Okonomi-yaki".

These combinations were subjected to measurement of blood glucose levels according to the above method for measuring blood glucose levels and then confirmed for the effect.

[Table 11]

| No. | No. 1 | No. 2 |
|---|---|---|
| Yeast extract added to sauce | No supplementation | NYP |
| n (number) | 10 | 8 |
| GI value | 47.7 | 41.5 |
| Standard deviation | 26.6 | 19.9 |

As shown in Table 11, the combination of Okonomi-yaki and the sauce supplemented with the yeast extract was found to have a GI value lower than that of the combination of Okonomi-yaki with the yeast extract-free sauce, and thus was revealed to more stably exert the effect of reducing the GI value (No. 2).

Example 7 Effect of reducing GI value exerted by combination of cooked rice and Dashi (Japanese soup stock) supplemented with yeast extract

In this Example, the effects of reducing GI values exerted by combinations of cooked rice and Dashi supplemented with yeast extracts were examined. Note that as the yeast extracts, two types of yeast extract; that is, a yeast extract extracted from yeast belonging to the genus *Saccharomyces* and a yeast extract extracted from *Torula yeast* were used.

(1) Five to eight male and female adults were randomly chosen as panelists.
(2) The following cooked rice was combined with Shirodashi (light-colored Japanese soup stock) supplemented with a yeast extract or Shirodashi, and then each panelist was instructed to eat such a combination.

Cooked rice: "Takitate Gohan Fukkura Tsuyadaki (well-rounded shiny freshly cooked rice)" (Tablemark Co., Ltd. Packed cooked rice (150 g) (the amount of carbohydrate: 49.6 g), microwaved at 500 W for 2 minutes);
Shirodashi supplemented with *Saccharomyces*-derived yeast extract (5 g): To "Kappou Shirodashi (restaurant-level Shirodashi)" (Registered Trademark) (Yamaki Co., Ltd., the amount of carbohydrate: 0.4 g), 1 g of a yeast extract "Yeast Extract 21-YK" (Fuji Foods Corporation. Hereinafter, referred to as "YK".) derived from yeast belonging to the genus *Saccharomyces* (*Saccharomyces cerevisiae*) was added, and then 45 g of water was added (yeast extract content: about 2 wt% of Shirodashi + water + yeast extract).

Shirodashi supplemented with *Torula yeast*-derived yeast extract (5 g): To Kappou Shirodashi (Registered Trademark) (Yamaki Co., Ltd., the amount of carbohydrate: 0.4 g), 1 g of Torula yeast (*Candida utilis*)-derived yeast extract "Yeast Extract 21-TF" (Fuji Foods Corporation. Hereinafter, referred to as "21TF".) was added, and 45g of water was added (yeast extract content: about 2 wt% of Shirodashi + water + yeast extract) or
Shirodashi (5 g): Kappou Shirodashi (Registered Trademark) (Yamaki Co., Ltd., the amount of carbohydrate: 0.4 g)
These combinations were subjected to measurement of blood glucose levels according to the above method for measuring blood glucose levels, and then confirmed for the effect.

[Table 12]

| No. | No. 1 | No. 2 | No. 3 |
|---|---|---|---|
| Yeast extract added to Shirodashi | No supplementation | YK (derived from yeast belonging to the genus *Saccharomyces*) | 21TF (derived from Torula yeast) |
| n (number) | 8 | 9 | 6 |
| GI value | 79.2 | 45.3 | 69.9 |

(continued)

| No. | No. 1 | No. 2 | No. 3 |
|---|---|---|---|
| Standard deviation | 24.9 | 25.1 | 29.9 |

As shown in Table 12, the combination of cooked rice and Shirodashi supplemented with the yeast extract derived from the genus *Saccharomyces* was found to have a GI value significantly lower than that of the combination of cooked rice with the yeast extract-free Shirodashi (No. 2). Furthermore, in the case of the Shirodashi supplemented with the Torula yeast-derived yeast extract, no significant effect of reducing the GI value was observed (No. 3).

**Claims**

1. A food composition for lowering a blood glucose level, containing a yeast extract of yeast belonging to the genus *Saccharomyces.*

2. A food composition for reducing a GI value, containing a yeast extract of yeast belonging to the genus *Saccharomyces.*

3. The food composition according to claim 1 or 2, containing the yeast extract in an amount of 0.001 wt% or more and 5.0 wt% or less in terms of the amount of the dry yeast extract.

4. The food composition according to any one of claims 1 to 3, wherein the yeast belonging to the genus *Saccharomyces* belongs to *Saccharomyces cerevisiae.*

5. A method for enhancing an effect of lowering a blood glucose level of a food composition, comprising incorporating a yeast extract of yeast belonging to the genus *Saccharomyces* into the food composition .

6. A method for reducing a GI value of a food composition, comprising incorporating a yeast extract of yeast belonging to the genus *Saccharomyces* into the food composition contains.

7. A combination of the food composition according to any one of claims 1 to 4 with a food composition containing grain as a main raw material.

8. The combination according to claim 7, wherein the food composition containing grain as a main raw material is rice, a noodle, a rice cake, bread, Okonomi-yaki, Tako-yaki, Monja-yaki, pizza, tortilla, dim sum, a Chinese steamed bun, a Korean pancake, a cereal food or a confectionery.

9. The combination according to claim 7 or 8, wherein the food composition containing grain as a main raw material is selected from the group consisting of Udon noodle, Hiyamugi, Soumen, Soba, pasta, Chinese noodles, and rice noodles.

10. The combination according to any one of claims 7 to 9, wherein the food composition containing grain as a main raw material is Udon noodle.

11. The combination according to any one of claims 7 to 10, wherein the food composition containing grain as a main raw material is produced by a method comprising a mixing step by reduced-pressure mixing.

12. The combination according to any one of claims 7 to 11, wherein the food composition according to any one of claims 1 to 4 is liquid.

13. The combination according to any one of claims 7 to 12, wherein the food composition containing grain as a main raw material contains a resistant starch.

14. The combination according to claim 13, wherein a content of the resistant starch is 0.6 wt% or more and less than 6 wt% of a total amount of powder raw materials.

15. The combination according to claim 13 or 14, wherein the resistant starch is obtained by subjecting a high-amylose corn starch to acid treatment, and has a peak molecular weight of $6\times10^3$ or more and $4\times10^4$ or less.

16. The combination according to any one of claims 7 to 15, used for lowering a blood glucose level.

17. The combination according to any one of claims 7 to 15, used for reducing a GI value.

18. A method for enhancing an effect of lowering a blood glucose level of both of the food composition according to any one of claims 1 to 4 and a food composition containing grain as a main raw material, comprising combining the food compositions.

19. A method for reducing a GI value of both of the food composition according to any one of claims 1 to 4 and a food composition containing grain as a main raw material, comprising combining the food compositions.

20. Noodles for lowering a blood glucose level, containing a resistant starch and produced by a method comprising a mixing step by reduced-pressure mixing.

21. A method for reducing a GI value of noodles, comprising incorporating a resistant starch into the noodles and producing the noodles by a method comprising a mixing step by reduced-pressure mixing.

Fig. 1

Fig. 2

Fig. 3

Fig. 4

Fig. 5

Fig. 6

Fig. 7

Fig. 8

Fig. 9

Fig. 10

# Fig. 11

# Fig. 12

Fraction 1

# Fig. 13

Fraction 2

# Fig. 14

Fraction 3

# Fig. 15

Fraction 4

# Fig. 16

# Fig. 17

# Fig. 18

## INTERNATIONAL SEARCH REPORT

| International application No. |
| --- |
| PCT/JP2017/015267 |

A. CLASSIFICATION OF SUBJECT MATTER
*A23L33/145*(2016.01)i, *A23L7/109*(2016.01)i, *A61K36/064*(2006.01)i, *A61P3/10*(2006.01)i

According to International Patent Classification (IPC) or to both national classification and IPC

B. FIELDS SEARCHED

Minimum documentation searched (classification system followed by classification symbols)
A23L33/145, A23L7/109, A61K36/064, A61P3/10

Documentation searched other than minimum documentation to the extent that such documents are included in the fields searched
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
| --- | --- | --- |
| X<br><br>Y | JP 2009-291076 A (Kaneka Corp.),<br>17 December 2009 (17.12.2009),<br>claims 1 to 6; paragraphs [0006], [0015] to [0016]; example 3<br>(Family: none) | 1,4-5,7-10, 16,18<br>1-21 |
| X<br><br>Y | JP 2005-082517 A (Asahi Denka Co., Ltd.),<br>31 March 2005 (31.03.2005),<br>claims 1 to 5; paragraphs [0005], [0014] to [0015], [0017], [0021] to [0022], [0039] to [0040], [0050], [0059]<br>(Family: none) | 1,4-5,7-10, 16,18<br>1-21 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 23 May 2017 (23.05.17) | 06 June 2017 (06.06.17) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

**INTERNATIONAL SEARCH REPORT**

| | International application No. |
|---|---|
| | PCT/JP2017/015267 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2015-500028 A (Lesaffre et Compagnie),<br>05 January 2015 (05.01.2015),<br>claims 1 to 11; paragraphs [0039], [0047],<br>[0049], [0059], [0065], [0067], [0122] to<br>[0130]<br>& US 2013/0071491 A1<br>claims 1 to 11; paragraphs [0053], [0061],<br>[0063], [0072], [0117] to [0122]<br>& WO 2013/084064 A2 & EP 2787840 A2<br>& CN 104023561 A | 1-8,13-14,<br>16-19<br>1-21 |
| X<br>Y | YANNI, A.E. et al., "Cr-enriched yeast: beyond<br>fibers for the management of postprandial<br>glycemic response to bread.", EUROPEAN JOURNAL<br>OF NUTRITION, 2016.02.25, Epub ahead of print<br>(pp.1-9), ISSN 1436-6215, <URL=http://link.<br>springer.com/article/10.1007/s00394-016-1190-4>,<br>particularly, page 6, left column, line 11 to<br>right column, line 28, page 7, right column,<br>lines 20 to 30, fig. 1 to 2 | 1-12,16-19<br>1-21 |
| X<br>Y | WEKSLER-ZANGEN, S. et al., "Glucose tolerance<br>factor extracted from yeast: oral insulin-<br>mimetic and insulin-potentiating agent: in vivo<br>and in vitro studies.", BRITISH JOURNAL OF<br>NUTRITION, 2012.09, Vol.108, No.5, pp.875-882,<br>ISSN 0007-1145, DOI: 10.1017/S0007114511006167,<br>particularly, abstract, page 881, right column,<br>lines 2 to 9, fig. 1 | 1-12,16-19<br>1-21 |
| X<br>Y | JP 2009-263655 A (ADEKA Corp.),<br>12 November 2009 (12.11.2009),<br>claims 4 to 5; paragraphs [0005] to [0009],<br>[0043] to [0046]<br>(Family: none) | 1-12,16-19<br>1-21 |
| Y | JP 2008-248082 A (Futamura Chemical Co., Ltd.),<br>16 October 2008 (16.10.2008),<br>claims 1 to 4; paragraphs [0002], [0037] to<br>[0038]<br>(Family: none) | 1-21 |
| A | JP 2006-022058 A (Takara Shuzo Co., Ltd.),<br>26 January 2006 (26.01.2006),<br>paragraph [0012]<br>(Family: none) | 1-21 |
| A | JP 2004-521127 A (Nutrition 21, Inc.),<br>15 July 2004 (15.07.2004),<br>claim 9; paragraphs [0053] to [0057], [0066]<br>& US 2002/0197331 A1<br>claim 9; paragraphs [0081] to [0085], [0094]<br>& WO 2002/067953 A2 & EP 1397148 A2 | 1-21 |

Form PCT/ISA/210 (continuation of second sheet) (January 2015)

**INTERNATIONAL SEARCH REPORT**

| International application No. |
|---|
| PCT/JP2017/015267 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-501829 A (Lesaffre et Compagnie), 01 February 2007 (01.02.2007), claims 1 to 19; paragraphs [0001] to [0007], [0026] to [0027], [0043] to [0045], [0051] to [0054] & US 2006/0257422 A1 claims 1 to 19; paragraphs [0001] to [0008], [0032] to [0037], [0060] to [0068], [0088] to [0096] & WO 2005/021015 A1 & EP 1663266 A1 & CN 1835761 A | 1-21 |
| A | CN 101658537 A (ANGEL YEAST CO., LTD.), 03 March 2010 (03.03.2010), page 8, lines 11 to 15; page 9, lines 9 to 11 (Family: none) | 1-21 |
| A | US 2013/0189298 A1 (TSAI, G.J.), 25 July 2013 (25.07.2013), claims 1, 8 & CN 103215194 A | 1-21 |
| A | JP 2011-084674 A (J-Oil Mills Inc.), 28 April 2011 (28.04.2011), paragraphs [0002], [0147] & US 2012/0196023 A1 paragraph [0002]; example 10 & WO 2011/045902 A1 & EP 2489682 A1 & CN 102574929 A | 14-15,20-21 |
| A | JP 2006-217813 A (National Food Research Institute), 24 August 2006 (24.08.2006), paragraphs [0001], [0005] to [0007], [0016] to [0018], [0053] (Family: none) | 14-15,20-21 |
| A | JP 2013-506427 A (University of Idaho), 28 February 2013 (28.02.2013), claims 1 to 5; paragraphs [0032] to [0037] & US 2011/0081475 A1 claims 1 to 4; paragraphs [0044] to [0049] & WO 2011/041702 A2 & EP 2482675 A1 | 14-15,20-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H0686379 B
- JP 2009291076 A
- JP 2011019402 A
- JP 2011084674 A
- JP H686379 B
- JP 4482611 B
- JP 2004180509 A

### Non-patent literature cited in the description

- **N. EDENS et al.** *J Nutr.,* June 2002, vol. 132 (6), 1141-1148
- **MATSUSHIMA M et al.** *Glycative Stress Research,* 2014, vol. 1, 53-59
- **KAWABATA A et al.** *Glycative Stress Research,* 2015, vol. 2 (2), 67-71
- **JENKINS, D. J. et al.** *Am. J. Clin. Nutr.,* 1981, vol. 34, 362-366
- **JENKINS, D.J. et al.** *Am. J. Clin. Nutr.,* 1981, vol. 34, 362-366